# EUROPEAN PATENT SPECIFICATION

(11) **EP 4 264 139 B1**
(45) Date of publication and mention of the grant of the patent: **11.09.2024**
(21) Application number: 21830504.3
(22) Date of filing: 02.12.2021
(51) Int. Cl.: F24F 3/167, H01L 21/673, H01L 21/677

(54) **CART-WINDOW ASSEMBLY**
FENSTER-WAGEN-BAUGRUPPE
ENSEMBLE CHARIOT-FENÊTRE

(30) Priority: 18.12.2020 IT 202000031400
(43) Date of publication of application: 25.10.2023
(73) Proprietor: Amira S.r.l., 20844 Truiggio (Monza Brianza) (IT)
(72) Inventor: DELMIGLIO, Angelo, 20844 Triuggio (Monza Brianza) (IT); DELMIGLIO, Mattia, 20844 Triuggio (Monza Brianza) (IT)
(74) Representative: Bellomia, Paolo
(86) International application number: PCT/IB2021/061218
(87) International publication number: WO 2022/130097

(56) References cited:
- WO-A1-2015/015995
- US-A- 4 964 899
- US-A- 5 277 654
- US-A- 5 401 212
- US-A- 5 431 599

## Description

### Technical field

The present invention relates to a cart-window assembly comprising a cart for moving material to/from a clean room and at least one window adapted to allow the communication between a dirty space and the clean room. In particular, the invention relates to a flowed cart and the related interface for the connection with a fixed window.

In a known manner, in laboratories and industries which treat pharmaceutical products, but also chemical, biomedical, cosmetic, food, electronic and similar products, the need arises to ensure that at least some areas of the plant are free of pollutants.

The invention is intended in particular for use in plants comprising at least one controlled contamination area, or *clean room*, i.e., an area with a very low amount of dusts and sterile from a microbiological point of view (hereinafter referred to in short as "clean room"). For this reason, the following description will make specific reference to this field of use. However, as those skilled in the art may well understand, the invention can also be usefully employed in different environments, for example in environments subject to less stringent constraints.

### Background art

In a known manner, one of the problems which constantly arises in systems comprising one or more clean rooms is that of correctly managing the transport of material from an unclassified external environment or an environment with a lower classification than that of the clean room (and therefore referred to in short as "dirty space") to a clean room.

For this purpose, solutions have been developed which are described below.

The flowed transfer carts referred to as LAF (*Laminar Air Flow*) are used to transport clean material from a first clean room to a second clean room, passing through a dirty space. These carts comprise a closed compartment into which air is blown which is sucked in from the external environment, typically filtered through HEPA (*High Efficiency Particulate Air*) filters, and reintroduced into the external environment. A double effect is thus obtained. On the one hand, it is ensured that an overpressure is established inside the compartment with respect to the external environment, so as to prevent pollutants from entering the compartment. On the other hand, the filtered air is blown in the form of a laminar flow in order to carry out a continuous washing of the surfaces, removing any suspended pollutants from the compartment and thus preventing it from contaminating the material loaded inside the cart. Thereby, the material, which was already clean at the start, remains clean throughout the journey, up to the second clean room.

It is another arising need to introduce material from a dirty space into a clean room, without thereby contaminating the clean room itself. For this purpose, the use of the so-called pass box, or material pass-through, is known, consisting of a compartment with a first door which opens towards the dirty space and a second door which opens towards the clean room. The pass box can then comprise a biodecontamination system which is suitable for the treatment of the contents of the compartment. The correct use of the pass box requires that, keeping the second door hermetically closed, a first operator in the dirty space opens the first door, introduces the contaminated material into the compartment and closes the first door. When both doors are hermetically closed, the biodecontamination cycle is carried out, for example by means of hydrogen peroxide vapor. Once the biodecontamination has been completed and the hydrogen peroxide residues inside the compartment have been disposed of by catalysis, a second operator inside the clean room can open the second door and take the material, now biodecontaminated and clean, so as to put it in the clean room without any risk of contamination.

While appreciated, such solutions are not without drawbacks.

In accordance with relatively recent regulations, there are areas inside the clean rooms (classified as areas A) which are subject to more stringent cleaning requirements than other areas (areas B). Typically, the area where the pass box is located and the area where the material processing machine is located are classified as areas A. In contrast, the adjacent areas of the clean room can be classified as areas B. The flowed LAF carts of the known type allow transporting clean material from one area A to another area A by passing through an area B. However, to do this the flowed LAF carts require that specific areas be set up in the areas A for the material loading and unloading operations, areas in which a special fixed LAF system ensures a laminar flow of clean air. The laminar flow of clean air, similar to what occurs inside the cart itself, prevents the contamination of the material while it is exposed during the loading and unloading operations. The fixed LAF plant is quite complex, and it is therefore preferable that the affected areas are identified from the beginning of the plant's construction. However, this is not always possible, for example for plants which are converted for different uses or for plants built before the issue of stringent regulations on the classification of areas A and B. In these cases the plant must be adapted to create new areas A with an adequate LAF plant in positions where they had not been envisaged. In this case, the necessary works involve significant costs and prolonged unavailability of the clean room and therefore an at least partial shutdown of the entire plant.

On the other hand, pass boxes are an expensive and inflexible solution. In fact, at least one pass box must be provided for each chamber. Furthermore, like the fixed LAF plant, the pass box also requires a rather complex and laborious installation and it is preferable to arrange it from the beginning of the plant's construction. The construction of a new pass box also requires works which involve prolonged unavailability of the clean room and therefore an at least partial shutdown of the entire plant.

Document WO2015/015995A1 describes an example of a cart for transferring materials, while document US5401212A describes an example of a transport system for transferring products between stations with different HEPA classes.

### Object of the invention

Therefore, it is the object of the present invention to overcome the drawbacks highlighted above in relation to the background art.

In particular, it is a task of the present invention to provide a flowed cart which does not require a fixed LAF plant near the pass box.

Furthermore, it is a task of the present invention to provide a flowed cart which, in addition to the peculiar operation thereof, allows maintaining the functions of the carts of known type.

Such an object and such tasks are achieved by a cart-window assembly according to claim 1 and the methods of claims 11-13.

### Brief description of the drawings

In order to better understand the invention and appreciate the further features and advantages thereof, some non-limiting exemplary embodiments thereof will be described below with reference to the accompanying drawings, in which:

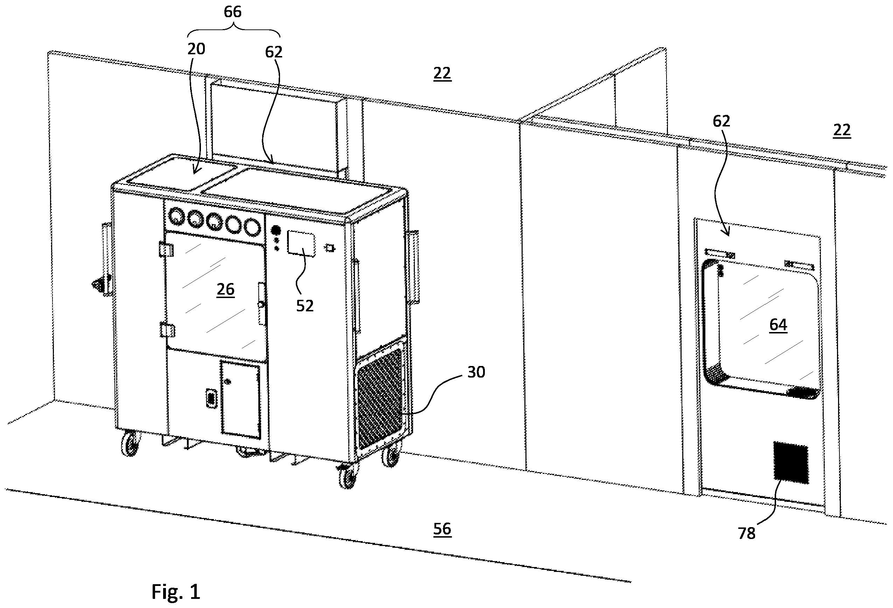

- figure 1 shows an axonometric view of a cart and a fixed communication window in accordance with the invention;
- figure 2 diagrammatically shows an embodiment of the cart in accordance with the invention, in an operating mode;
- figure 3 diagrammatically shows another embodiment of the cart in accordance with the invention, in another operating mode;
- figure 4 diagrammatically shows the embodiment in figure 3, in another operating mode;
- figure 5 diagrammatically shows the embodiment in figure 3, in another operating mode;
- figure 6 shows an axonometric view of an embodiment of the cart in accordance with the invention, in which some parts have been removed;
- figure 7 shows a plan view similar to that in figure 1, in which some parts of the cart have been removed;
- figure 8 shows a view of the section made along the line VIII-VIII in figure 7;
- figures 9.a and 9.b show two different configurations of the section made along the line IX-IX in figure 8; and
- figures 10.a and 10.b show two different configurations of the detail indicated with X in figure 9.

### Detailed description of preferred embodiments of the invention

In the context of the present discussion, some terminological conventions have been adopted in order to make reading easier and more fluent. Such terminological conventions are clarified below with reference to the accompanying drawings.

The invention relates to a movable cart in an industrial environment. The expression "fixed" refers hereinafter to elements of the environment, as opposed to the elements of the cart which are movable therewith.

The expression "clean room" means a constantly controlled environment which meets certain predefined requirements and which therefore has a very low amount of dust and is sterile from a microbiological point of view. The expression "dirty space" means an environment outside a clean room and for which there is no requirement or there are less stringent requirements than those of the clean room.

The expression "HP mixture" hereinafter refers to a mixture of gas, for example air, and hydrogen peroxide (H₂O₂ - *Hydrogen Peroxide*), adapted to obtain a biodecontamination effect on the surfaces with which it comes into contact.

The expression "HP apparatus" hereinafter refers to a device adapted to generate a flow of HP mixture. The HP apparatus can utilize different technologies: for example, it can utilize technologies based on the use of hydrogen peroxide in the vapor state such as VHP (*Vaporized Hydrogen Peroxide*), HPV (*Hydrogen Peroxide Vapor*), VPHP (*Vapor Phase Hydrogen Peroxide*) technologies, or it can utilize technologies based on ionized hydrogen peroxide such as IHP (*Ionized Hydrogen Peroxide*) or HPE technologies, or it can utilize so-called *dry fog* technologies such as spraying or atomizing hydrogen peroxide.

Within the scope of the invention, paths are defined within which a fluid moves in a unique direction. In relation to such a direction, the expressions "upstream", "before" or the like are uniquely defined with respect to the expressions "downstream", "after" or the like.

The invention relates to a cart-window assembly 66 comprising a cart 20 for moving material to/from a clean room 22 and at least one window 62 adapted to allow the communication between a dirty space 56 and the clean room 22.

In particular, the cart 20 for moving material from/to a clean room 22 comprises:
- a compartment 24;
- at least one protected door 28 adapted to alternatively allow accessing the compartment 24 and hermetically closing the compartment 24; and
- a ventilation system 30 adapted to create an air flow inside the compartment 24;

in which the cart 20 further comprises first interface means 60.1 surrounding the protected door 28, in which the first interface means 60.1 are complementary to second interface means 60.2 of a window 62 adapted to allow the communication between the dirty space 56 and the clean room 22.

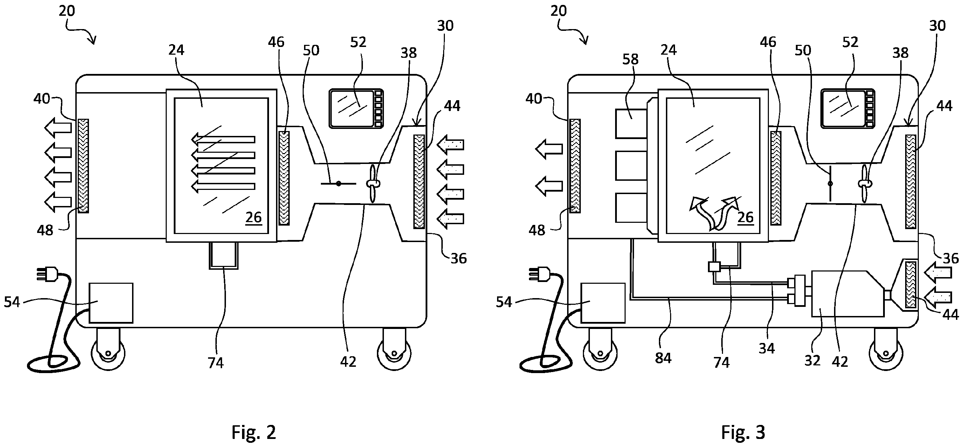

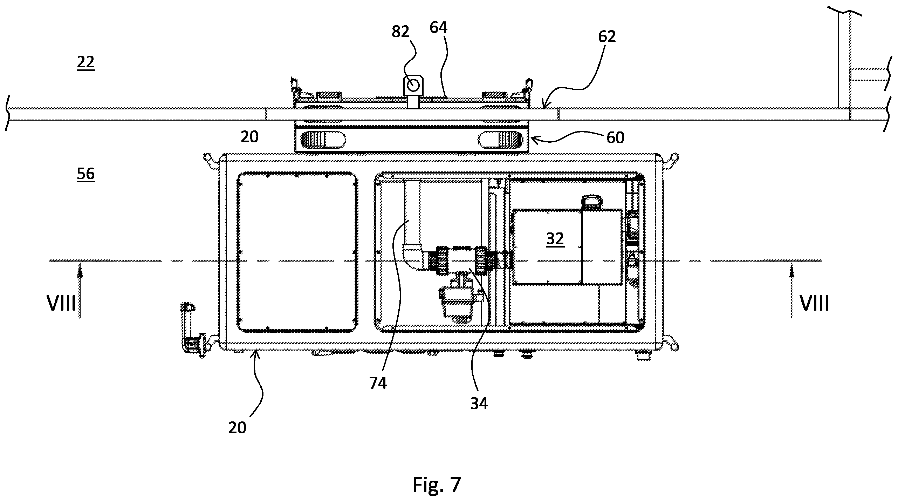

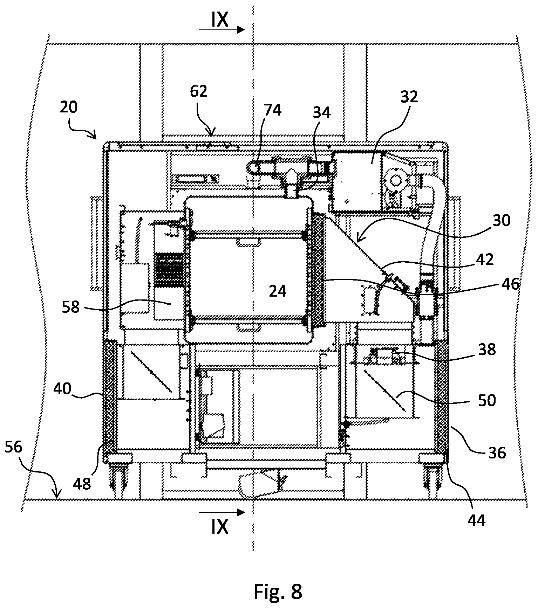

In accordance with some embodiments (for example those in figures 3 to 9), the cart 20 further comprises a biodecontamination system. The biodecontamination system can comprise an ultraviolet lamp. For example, in a manner known per se, the lamp can emit a so-called ultraviolet germicidal irradiation (UVGI), i.e., an ultraviolet radiation with wavelengths comprised in the UV-C band (between 100 and 280 nanometers). Alternatively or in addition, the biodecontamination system can comprise an HP apparatus 32 and a first conduit 34 adapted to introduce a flow of hydrogen peroxide-based HP mixture into the compartment 24.

Preferably, the cart 20 can further comprise an exposed door 26, also adapted to alternatively allow accessing the compartment 24 and hermetically closing the compartment 24. From the following description, those skilled in the art can well understand how both doors 26, 28 are exposed in the same identical manner in many conditions of use. However, the possibility of obtaining a protection for one of the two doors 26, 28 is one of the technical features of the cart 20 which allows making the most of the advantages of the invention.

In accordance with some embodiments, the ventilation system 30 is adapted to create a laminar air flow.

The ventilation system 30 can be similar per se to those mounted on board the flowed LAF (*Laminar Air Flow*) carts of a known type. Preferably the ventilation system 30 comprises a first opening 36 for the suction of air from the outside, one or more filters (described in greater detail below), a fan 38, a second opening 40 for expelling the air towards the outside, and a series of channels 42 adapted to connect the first opening 36 to the fan 38 and then to the compartment 24 of the cart 20, and adapted to connect the compartment 24 to the second opening 40. Preferably, the ventilation system 30 comprises one or more of the following filters:
- a suction filter 44, placed at the first opening 36, adapted to intercept the air flow sucked in from the outside by the ventilation system 30;
- an introduction filter 46, placed near the compartment 24, adapted to intercept the air flow which is blown into the compartment 24; and/or
- an exhaust filter 48, placed at the second opening 40, adapted to intercept the air flow which is expelled to the outside.

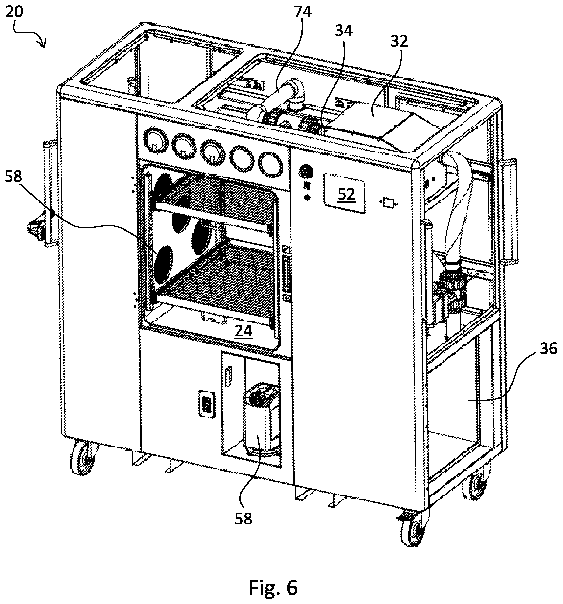

Furthermore, in accordance with some embodiments (for example that in figures 3 to 5), the cart 20 comprises a second suction filter 44, adapted to intercept the air flow sucked from the outside by the HP apparatus 32. In accordance with other embodiments (for example that in figures 6 to 8), the cart 20 comprises a single suction filter 44 which serves both the ventilation system 30 and the HP apparatus 32.

Preferably, one or more of the filters listed above are so-called absolute filters, for example they can be said HEPA (*High Efficiency Particulate Air*) or ULPA (*Ultra Low Penetration Air*) filters based on the classification defined by the UNI EN 1822 standards. In particular, HEPA filters have a filtration efficiency between 85% and 99.995% depending on the class to which they belong, while ULPA filters have a filtration efficiency between 99.9995% and 99.999995% depending on the class to which they belong. Advantageously, the ventilation system 30 further comprises a shutter 50, preferably motorized, adapted to close the channel 42 upstream of the compartment 24.

Preferably, the fan 38 is a centrifugal fan.

Advantageously, the cart 20 comprises a control panel 52 which allows one or more of the following functions to be performed:
- controlling and/or programming the times and operating modes of the cart 20;
- recording the operating parameters, for example during a use cycle;
- checking the status of the cart 20 and reporting malfunctions, breakdowns or the need for routine maintenance.

Preferably, the cart 20 comprises an electrical power supply system 54, adapted to power the devices on board the cart 20. The power supply system 54 preferably comprises a connection to the mains and/or a battery or an uninterruptible power supply which allows the on-board devices to be powered even while the cart 20 is disconnected from the mains, typically when it is in motion. A device on board the cart 20 which must be powered is certainly the fan 38 of the ventilation system 30 but, in accordance with the various embodiments, other devices may also require an energy supply.

In a manner known per se, the ventilation system 30 is adapted to achieve two purposes. Firstly, the ventilation system 30 ensures that an overpressure with respect to the external environment is established inside the compartment 24. Furthermore, the ventilation system 30 can be adapted to blow air in the form of a laminar flow inside the compartment 24.

These features allow the cart 20 to have the operating mode diagrammatically shown in figure 2. In accordance with such a mode, the cart 20 of the invention functions exactly like a flowed LAF cart 20 of a known type. In particular, a laminar flow of clean air is ensured inside the compartment 24 which prevents any pollutant suspended in the air from contaminating the material stored inside the compartment 24. Thereby the cart 20 of the invention can be used to transport clean material from a first clean room 22 to a second clean room 22, passing through a dirty space 56.

In accordance with some embodiments, the HP apparatus 32 is a generator of the VPHP (*Vapor Phase Hydrogen Peroxide*) type, known per se. In particular, a VPHP generator is adapted, starting from hydrogen peroxide in the liquid phase, to generate hydrogen peroxide in the vapor phase and thus to obtain the HP mixture. Advantageously, the cart 20 comprises a tank 58 of liquid phase hydrogen peroxide from which the HP apparatus 32 draws to obtain the vapor phase hydrogen peroxide. Preferably, the HP apparatus 32 is also adapted to suck an air flow from the outside, for mixing a predetermined flow rate of hydrogen peroxide vapor with a predetermined flow rate of air, so as to obtain the HP mixture. Preferably, the cart 20 of the invention comprises a first conduit 34 which connects the outlet of the HP apparatus 32 inside the compartment 24, so that the HP mixture can be introduced into the compartment 24 itself.

A particularly efficient VPHP generator 32 which is particularly suitable for use in the cart 20 of the invention is described in European patent application EP 3 669 896, filed by the same Applicant.

Preferably, the cart 20 further comprises one or more main catalysts 58, adapted to ensure the splitting of the hydrogen peroxide molecules (H₂O₂) into water (H₂O) and oxygen (O₂). The main catalysts 58, known per se, preferably comprise metal grids with a coating of heavy metals such as mixtures of palladium, iridium, chromium, and the like. Advantageously, the main catalysts 58 are placed along the ventilation system 30, downstream of the compartment 24 and preferably upstream of the second opening 40 for expelling the air towards the outside.

The technical features of these embodiments allow the cart 20 to have the operating mode diagrammatically shown in figure 3. In accordance with such a method, the cart 20 of the invention allows carrying out a biodecontamination process of the compartment 24 and the material stored therein. In particular, the HP device 32 produces the HP mixture which is fed into the compartment 24 by the first conduit 34. The shutter 50 is closed to prevent the HP mixture from accidentally passing through the ventilation system 30 in the opposite direction. Since hydrogen peroxide is an irritant, the ventilation requires the flow of HP mixture to be passed through the main catalysts 58 before being expelled, so that the hydrogen peroxide is split into water and oxygen and only harmless substances are expelled outside the cart 20. At the end of the biodecontamination cycle, the cart 20 should be brought into an operating mode similar to that in figure 2 with the only difference that in this case the main catalysts 58 are also involved. In other words, the HP apparatus 32 is deactivated and therefore the HP mixture is no longer fed inside the compartment 24. Furthermore, the shutter 50 is opened and the fan 38 is activated. Thereby, the forced ventilation inside the compartment 24 allows any residual hydrogen peroxide to be removed and passed through the main catalysts 58, to ensure that the compartment 24 can be opened in total safety.

The assembly 66 further comprises a fixed window 62 adapted to allow the communication between the dirty space 56 and the clean room 22, in which the window 62 comprises a panel 64 which can alternatively be open or sealingly closed, and second interface means 60.2 extending along the periphery of the panel 64 and which are complementary to the first interface means 60.1 arranged on board the cart 20 in accordance with the invention.

Advantageously, the panel 64 opens from the side of the window 62 opposite to the side on which the second interface means 60.2 are arranged. In accordance with some embodiments, the window 62 is mounted so that the panel 64 opens towards the clean room 22 and the second interface means 60.2 face the dirty space 56. In accordance with other embodiments, vice versa, the window 62 is mounted so that the panel 64 opens towards the dirty space 56 and the second interface means 60.2 face the clean room 22.

Advantageously, the cart 20 thus comprises first interface means 60.1 surrounding the protected door 28 and the window 62 comprises second interface means 60.2 extending along the periphery of the panel 64, in which the first interface means 60.1 and the second interface means 60.2 are mutually complementary and are referred to as interface means 60 as a whole.

In the assembly 66, the interface means 60 allow, if necessary (i.e., reversibly), the cart 20 to be firmly coupled to the window 62 so as to define a sealingly closed gap 68 between the two. In particular, when the interface means 60 are fully activated, the gap 68 is sealingly closed towards the compartment 24 of the cart 20, towards the dirty space 56, and towards the clean room 22.

Preferably, the interface means 60 comprise sensors adapted to detect the correct position of the cart 20 with respect to the fixed window 62. The sensors can be positioned on the cart 20, on the window 62 or, preferably, on both. Preferably, the interface means 60 comprise electromagnets adapted to be activated to lock the cart 20 in the correct position with respect to the fixed window 62. The electromagnets can be positioned on the cart 20, on the window 62, or on both.

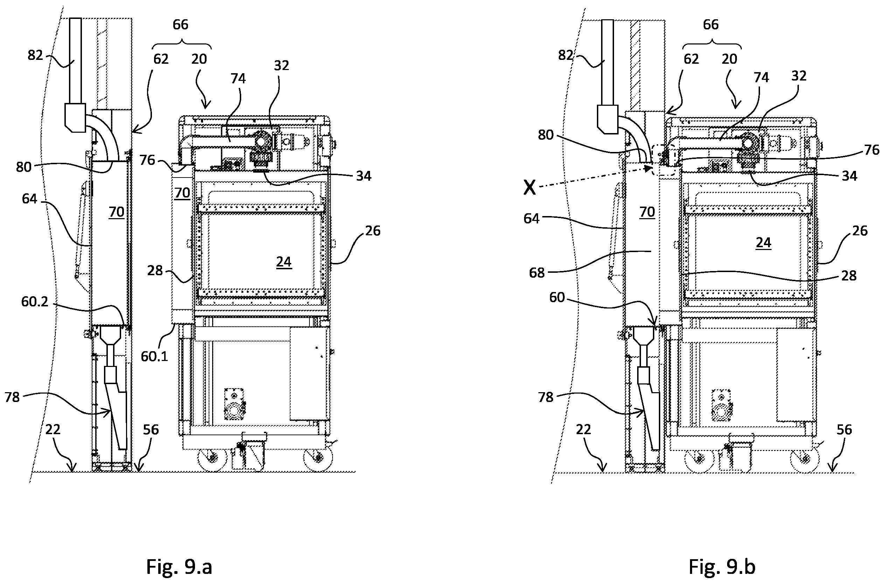

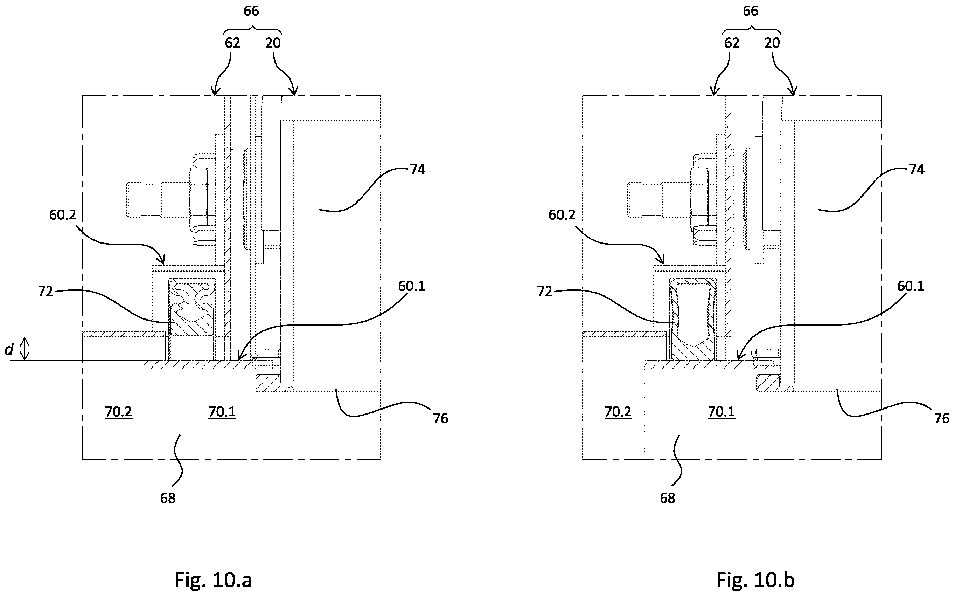

A particular embodiment of the interface means 60 is described below with reference to figures 9 and 10. In accordance with such an embodiment, the interface means 60 comprise a pair of sleeve flanges 70 complementary to each other. A first sleeve flange 70.1 is mounted on the cart 20, so as to surround the protected door 28, and a second sleeve flange 70.2 is mounted on the window 62, so as to surround the panel 64. The two sleeve flanges 70 are complementary to each other, meaning that one sleeve flange (for example the second sleeve flange 70.2 mounted on the window 62) can accommodate the other sleeve flange therein (in the example the first sleeve flange 70.1 mounted on the cart 20). In particular, the two sleeve flanges 70 have such dimensions that, while the cart 20 is in the correct position with respect to the fixed window 62, they remain at a distance d one from the other on all sides (see the detail in figure 10.a). The distance d can be between about 1 mm and about 50 mm, preferably between about 5 mm and about 30 mm.

Preferably, the interface means 60 further comprise a gasket 72. In accordance with the embodiment shown in the figures, the gasket 72 is of the pneumatically actuated type, known per se, and is adapted to bridge the distance d between the two sleeve flanges 70 so as to ensure the closure of the gap 68 (see the detail in figure 10.b). Furthermore, the actuation of the gasket 72, for example pneumatic, ensures that it is compressed, so as to ensure the sealing closure of the gap 68.

In the embodiment shown in figure 10, the gasket 72 is arranged on the second interface means 60.2 of the window 62. Therefore, in this case the window 62 preferably comprises a system for activating the gasket 72, for example a pneumatic system which makes compressed air available. In other embodiments (not shown), the gasket 72 can be mounted on the first interface means 60.1 of the cart 20. Therefore, in this case the cart 20 can comprise a system for activating the gasket 72, for example a pneumatic system which makes compressed air available. In still other embodiments, it is possible to include a pneumatic connection between the cart 20 and the window 62, so that a fixed system can feed a gasket 72 on board the cart 20 or, vice versa, so that a system on board the cart 20 can feed a gasket 72 arranged on the fixed window 62.

As those skilled in the art can well understand, the embodiment of the interface means 60 described above is only one among many possible ones. For example, in accordance with other embodiments (not shown), the interface means 60 can comprise plates designed to come into contact with each other and an electromagnetic coupling. The activation of the electromagnetic coupling, in addition to ensuring that the cart 20 remains firmly in position, also ensures that a gasket 72 is adequately compressed to obtain the seal.

Preferably, the window 62 can comprise a mouth 80 between the panel 64 and the second interface means 60.2, connected to a fixed HP system 82. It should be noted that, in accordance with the terminological convention adopted in the description, the HP system 82 is defined as "fixed", meaning thereby that it is not mounted on board the cart 20. However, as those skilled in the art can well understand, the HP system 82 can in turn be a movable device to connect to the mouth 80 only when necessary, without thereby modifying the invention in any manner. In any case, when the cart 20 is firmly coupled to the window 62 and the interface means 60 are activated, the mouth 80 is inside the gap 68. Thereby, it is possible to introduce the HP mixture produced by the fixed HP system 82 inside the gap 68 itself.

Preferably, the cart 20 comprises a second conduit 74 connecting the interior of the compartment 24 to a mouth 76 outside the compartment 24, between the protected door 28 and the first interface means 60.1 (figure 9). When the cart 20 is firmly coupled to the window 62 and the interface means 60 are activated, the mouth 76 is inside the gap 68.

In accordance with some embodiments, the second conduit 74 connects the mouth 76 to the first conduit 34. Thereby, the second conduit 74 allows, if required, connecting the outlet of the HP apparatus 32 to the mouth 76 and/or the mouth 76 to the compartment 24.

Thereby, by means of the fixed mouth 80 of the window 62 and/or through the mouth 76 of the cart 20, it is possible to introduce the HP mixture into the gap 68 itself.

Preferably, the window 62 comprises a second ventilation system 78 of the gap 68, in turn comprising an exhaust filter and a fixed catalyst, both similar to the corresponding components of the cart 20 described above. In order obtain the forced circulation of the air inside the gap 68 and the second ventilation system 78, it is possible to utilize the flow arriving from the HP apparatus 32. Alternatively or in addition, it is possible to arrange a second fixed fan or another ventilation device (such as a source of compressed air).

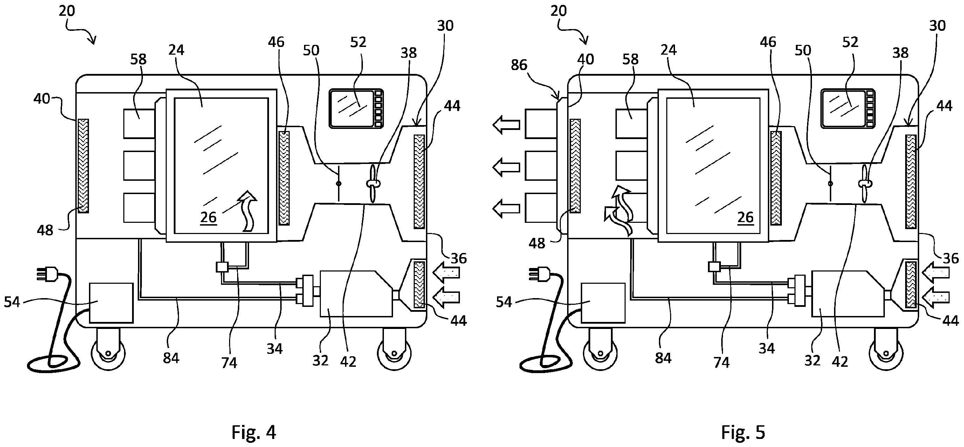

These features allow the cart 20 to have the operating mode diagrammatically shown in figure 4. In accordance with such a mode, the biodecontamination of the gap 68 can be ensured, introducing the HP mixture therein, similarly to what was described above in relation to the compartment 24. At the end of the biodecontamination treatment of the gap 68, the second ventilation system 78 and the respective fixed catalysts allow removing any residual hydrogen peroxide vapor from the gap 68 so as to ensure the safety of the operator who must open the panel 64.

Advantageously, the cart 20 can comprise a third conduit 84 connecting the outlet of the HP apparatus 32 to a point inside the ventilation system 30, downstream of the main catalysts 58 and preferably upstream of the second opening 40 for the expulsion of the air towards the outside. In some embodiments, such as that diagrammatically shown in figures 3 to 5, the third conduit 84 is permanently arranged on board the cart 20. In other embodiments, such as that shown in figures 6 to 9, the third conduit 84 can be assembled and disassembled if required. Advantageously, the cart 20 further comprises a removable accessory 86 comprising an air manifold and one or more accessory catalysts which are completely similar to the main catalysts 58 described above. The manifold is adapted to be coupled to the second opening 40 in a removable manner for expelling the air, so as to intercept the flow of gas output from the ventilation system 30 of the cart 20.

These features allow the cart 20 to have the operating mode diagrammatically shown in figure 5. In accordance with such a mode, the cart 20 also allows a biodecontamination cycle to be carried out on the second opening 40 for expelling the air towards the outside and in particular on the possible exhaust filter 48 arranged thereat. This portion of the ventilation system 30, being located downstream of the main catalysts 58, is never reached by the hydrogen peroxide and is therefore exposed to phenomena of microbiological contamination. The third conduit 84 allows the HP mixture to be fed immediately upstream of the second opening 40 and the possible exhaust filter 48 while the removable accessory 86, with the accessory catalysts thereof, ensures the safety of the operators.

The different modes of use described above with reference to figures 2 to 5 allow the cart 20 of the invention to perform different types of missions which are described below.

One possible mission is to transfer already cleaned material from a first clean room 22 to a second clean room 22 passing through a dirty space 56. In order to carry out this mission safely, it is possible to arrange a cart 20 in accordance with the invention in the dirty space 56; arrange the first clean room 22 with a first window 62 and the second clean room 22 with a second window 62, in accordance with what is described above. In particular, the window 62 must be arranged so that the second interface means 60.2 face the dirty space 56 and the panel 64 opens towards the clean room 22. It is then possible to carry out the following steps:
- coupling the cart 20 to the first window 62 of the first clean room 22 by the interface means 60 so as to form a first gap 68 (figures 9.b and 10.a);
- sealingly closing the first gap 68 (figure 10.b);
- biodecontaminating the first gap 68;
- eliminating the hydrogen peroxide residues from the first gap 68;
- opening the panel 64 of the first window 62;
- opening the protected door 28 of the cart 20;
- loading the clean material into the compartment 24;
- sealingly closing the protected door 28;
- sealingly closing the panel 64 of the first window 62;
- uncoupling the cart 20 from the first window 62 of the first clean room 22;
- transporting the cart 20 close to the second clean room 22 (figure 9.a);
- coupling the cart 20 to the second window 62 of the second clean room 22 by the interface means 60 so as to form a second gap 68 (figures 9.b and 10.a);
- sealingly closing the second gap 68 (figure 10.b);
- biodecontaminating the second gap 68;
- eliminating the hydrogen peroxide residues from the second gap 68;
- opening the panel 64 of the second window 62;
- opening the protected door 28 of the cart 20;
- unloading the clean material from the compartment 24;
- sealingly closing the protected door 28;
- sealingly closing the panel 64 of the second window 62; and
- uncoupling the cart 20 from the second window 62 of the second clean room 22.

It should be noted that in this type of mission, the steps of biodecontaminating the gaps 68 can be completed using both the HP system 82 of the window 62, and using the optional HP apparatus 32 of the cart 20, by virtue of the presence of the second conduit 74.

It should further be noted that in this type of mission, between the step of uncoupling the cart 20 from the first window 62 of the first clean room 22 and the step of coupling the cart 20 to the second window 62 of the second clean room 22, an indefinite period of time can elapse. In fact, the cart 20 can be positioned in a waiting area where, ensuring the power supply of the on-board devices, the cart 20 can remain in total safety for an indefinite time.

Another possible mission is to transfer dirty material from a dirty space 56 to a clean room 22. In order to carry out this mission safely, it is possible to arrange a cart 20 in accordance with the invention and comprising the second conduit 74 in the dirty space 56; and arrange the clean chamber 22 with a window 62 in accordance with what is described above. In particular, the window 62 must be arranged so that the second interface means 60.2 face the dirty space 56 and the panel 64 opens towards the clean room 22. It is then possible to carry out the following steps:
- opening a door of the cart 20 (indifferently the protected door 28 or the exposed door 26);
- loading the dirty material into the compartment 24;
- sealingly closing the door;
- coupling the cart 20 to the window 62 of the clean room 22 by the interface means 60 so as to form a gap 68 (figures 9.b and 10.a);
- sealingly closing the gap 68 (figure 10.b);
- biodecontaminating the gap 68, the interior of the compartment 24 of the cart 20 and the material;
- eliminating the hydrogen peroxide residues from the gap 68 and the compartment 24;
- opening the panel 64 of the window 62;
- opening the protected door 28 of the cart 20;
- unloading the clean material from the compartment 24;
- sealingly closing the protected door 28;
- sealingly closing the panel 64 of the window 62; and
- uncoupling the cart 20 from the window 62 of the clean room 22.

It should be noted that in this mission, the cart 20 of the invention behaves exactly like a movable pass box, i.e., a pass box which can be applied from time to time to the window 62 of the clean room 22 which requires it.

It should also be noted that the step of biodecontaminating the gap 68, the interior of the compartment 24 of the cart 20 and the material can be completed either by using the HP system 82 of the window 62, or by using the optional HP apparatus 32 of the cart 20, by virtue of the presence of the second conduit 74 connecting the interior of the compartment 24 to the mouth 76. In this case it may be preferable to provide auxiliary fans (not shown) to facilitate the circulation of the HP mixture between the compartment 24 and the gap 68 and finally to convey it towards the catalysts.

A further possible mission is to transfer dirty material from a dirty space 56 to a clean room 22 and then move it safely inside the clean room 22, typically between two areas A passing through an area B.

In order to carry out this mission safely, it is possible to arrange a cart 20 in accordance with the invention and comprising the second conduit 74 in the clean room 22; and arrange the clean chamber 22 with a window 62 in accordance with what is described above. In particular, the window 62 must be arranged so that the second interface means 60.2 face the clean room 22 and the panel 64 opens towards the dirty space 56. It is then possible to carry out the following steps:
- coupling the cart 20 to the window 62 of the clean room 22 by the interface means 60 so as to form a gap 68 (figures 9.b and 10.a);
- sealingly closing the gap 68 (figure 10.b);
- opening the panel 64 of the window 62;
- opening the protected door 28 of the cart 20;
- loading the dirty material into the compartment 24;
- closing the protected door 28;
- sealingly closing the panel 64 of the window 62;
- biodecontaminating the interior of the compartment 24 of the cart 20, the material, and the gap 68;
- eliminating the hydrogen peroxide residues from the compartment 24 and the gap 68;
- uncoupling the cart 20 from the window 62;
- moving the cart 20 inside the clean room 22;
- opening a door of the cart 20; and
- unloading the clean material from the compartment 24.

It should be noted that in this mission, the cart 20 of the invention behaves like a pass box and then allows the clean and biodecontaminated material to be transported in complete safety also through the areas B of the clean room 22.

Therefore, it should be noted how the cart 20 of the invention greatly simplifies the management of accesses to the clean rooms. In fact, by adopting at least one cart 20 in accordance with the invention, it is no longer necessary to provide each clean room 22 with a pass box thereof nor with a fixed LAF plant. On the contrary, it is sufficient to provide a simple window 62 in accordance with what is described above for each clean room 22. The window 62 is much simpler than the pass box and the fixed LAF plant. In the most essential version thereof, the window 62 comprises only the interface means 60 and the watertight panel 64.

Therefore, the provision of such a window 62 does not require complex work and reduces the unavailable time of the clean room 22 and partial shutdown of the system to a minimum.

The foregoing description elaborates on the technical features which distinguish the invention from the solutions of the known art. For all the other features, which may be common to the known art and the invention, reference should be made to the introduction where the prior art is described and commented.

As those skilled in the art can well understand, the invention allows overcoming the drawbacks highlighted above with reference to the background art.

In particular, the present invention provides a flowed cart 20 which does not require a fixed LAF plant.

Furthermore, the present invention provides a flowed cart 20 which, in addition to the peculiar operations thereof, allows maintaining the functions of the carts of known type.

It is apparent that the specific features are described in relation to different embodiments of the invention with an illustrative and non-limiting intent. Obviously, those skilled in the art can make further changes and variations to the present invention, in order to meet contingent and specific needs. For example, the technical features described in relation to an embodiment of the invention can be extrapolated therefrom and applied to other embodiments of the invention. Such changes and variations are however contained within the scope of protection of the invention, as defined by the following claims.

## Claims

1. A cart-window assembly (66) comprising a cart (20) for moving material to/from a clean room (22) and at least one window (62) adapted to allow the communication between a dirty space (56) and said clean room (22);
wherein said cart (20) comprises:
- a compartment (24),
- at least one protected door (28) adapted to alternatively allow accessing the compartment (24) and hermetically closing the compartment (24),
- a ventilation system (30), and
- first interface means (60.1) surrounding the protected door (28);
wherein said window (62) comprises:
- a panel (64) which can alternatively be open or sealingly closed and
- second interface means (60.2) extending along the periphery of the panel (64);
**characterized in that** the first interface means (60.1) and the second interface means (60.2) are mutually complementary and allow the cart (20) to be firmly coupled to the window (62) in a reversible manner, so as to define a gap (68) therebetween which is sealingly closed.

2. An assembly (66) according to claim 1, wherein the ventilation system (30) of the cart (20) is adapted to create a laminar air flow inside the compartment (24).

3. An assembly (66) according to claim 1 or 2, wherein the cart (20) further comprises a biodecontamination system.

4. An assembly (66) according to claim 3, wherein the biodecontamination system comprises a lamp adapted to emit an ultraviolet germicidal irradiation (UVGI).

5. An assembly (66) according to claim 3, wherein the biodecontamination system comprises an HP apparatus (32) and a first conduit (34) adapted to introduce a flow of hydrogen peroxide-based HP mixture into the compartment (24).

6. An assembly (66) according to claim 5, wherein the cart (20) further comprises one or more main catalysts (58) placed along the ventilation system (30), downstream of the compartment (24) and adapted to ensure the splitting of the hydrogen peroxide (H₂O₂) molecules into water (H₂O) and oxygen (02).

7. An assembly (66) according to any one of the preceding claims, wherein the cart (20) comprises a second conduit (74) connecting the interior of the compartment (24) to a mouth (76) outside the compartment (24), between the protected door (28) and the first interface means (60.1).

8. An assembly (66) according to claim 5 or 6, wherein the cart (20) further comprises:
- a third conduit (84) connecting the outlet of the HP apparatus (32) to a point inside the ventilation system (30), downstream of the main catalysts (58); and
- a removable accessory (86) comprising an air manifold and one or more ancillary catalysts.

9. An assembly (66) according to any one of the preceding claims, wherein the window (62) further comprises a mouth (80) between the panel (64) and the second interface means (60.2), connected to a fixed HP system (82).

10. An assembly (66) according to claim 9, wherein the window (62) further comprises a second ventilation system (78) comprising an exhaust filter and fixed catalysts.

11. A method for transferring clean material from a first clean room (22) to a second clean room (22) passing through a dirty space (56), comprising the steps of:
- providing a cart (20) of an assembly (66) according to one or more of claims 1 to 10 in the dirty space (56);
- providing the first clean room (22) with a first window (62) of an assembly (66) according to one or more of claims 1 to 10;
- providing the second clean room (22) with a second window (62) of an assembly (66) according to one or more of claims 1 to 10;
- coupling the cart (20) to the first window (62) by the interface means (60) so as to form a first gap (68);
- sealingly closing the first gap (68);
- biodecontaminating the first gap (68);
- eliminating the hydrogen peroxide residues from the first gap (68);
- opening the panel (64) of the first window (62);
- opening the protected door (28) of the cart (20);
- loading the clean material into the compartment (24);
- sealingly closing the protected door (28);
- sealingly closing the panel (64) of the first window (62);
- uncoupling the cart (20) from the first window (62) of the first clean room (22);
- transporting the cart (20) close to the second clean room (22);
- coupling the cart (20) to the second window (62) by the interface means (60) so as to form a second gap (68);
- sealingly closing the second gap (68);
- biodecontaminating the second gap (68);
- eliminating the hydrogen peroxide residues from the second gap (68);
- opening the panel (64) of the second window (62);
- opening the protected door (28) of the cart (20);
- unloading the clean material from the compartment (24);
- sealingly closing the protected door (28);
- sealingly closing the panel (64) of the second window (62); and
- uncoupling the cart (20) from the second window (62) of the second clean room (22).

12. A method for transferring dirty material from a dirty space (56) to a clean room (22), comprising the steps of:
- providing a cart (20) of an assembly (66) according to one or more of claims 1 to 10 in the dirty space (56);
- providing the clean room (22) with a window (62) of an assembly (66) according to one or more of claims 1 to 10;
- opening a door (26, 28) of the cart (20);
- loading the dirty material into the compartment (24);
- sealingly closing the door (26, 28);
- coupling the cart (20) to the window (62) of the clean room (22) by the interface means (60) so as to form a gap (68);
- sealingly closing the gap (68);
- biodecontaminating the gap (68), the interior of the compartment (24), and the material;
- eliminating the hydrogen peroxide residues from the gap (68) and the compartment (24);
- opening the panel (64) of the window (62);
- opening the protected door (28) of the cart (20);
- unloading the clean material from the compartment (24);
- sealingly closing the protected door (28);
- sealingly closing the panel (64) of the window (62); and
- uncoupling the cart (20) from the window (62) of the clean room (22).

13. A method for transferring dirty material from a dirty space (56) to a clean room (22), comprising the steps of:
- providing a cart (20) of an assembly (66) according to one or more of claims 1 to 10 in the clean room (22);
- providing the clean room (22) with a window (62) of an assembly (66) according to one or more of claims 1 to 10;
- coupling the cart (20) to the window (62) of the clean room (22) by the interface means (60) so as to form a gap (68);
- sealingly closing the gap (68);
- opening the panel (64) of the window (62);
- opening the protected door (28) of the cart (20);
- loading the dirty material into the compartment (24);
- closing the protected door (28) of the cart (20);
- sealingly closing the panel (64) of the window (62);
- biodecontaminating the interior of the compartment (24), the material, and the gap (68);
- eliminating the hydrogen peroxide residues from the compartment (24) and the gap (68);
- uncoupling the cart (20) from the window (62);
- moving the cart (20) inside the clean room (22);
- opening a door (26, 28) of the cart (20); and
- unloading the clean material from the compartment (24).

## Patentansprüche

1. Fenster-Wagen-Baugruppe (66), umfassend einen Wagen (20) zum Bewegen von Material zu/von einem Reinraum (22) und mindestens ein Fenster (62), das ausgelegt ist, um die Kommunikation zwischen einem verschmutzten Bereich (56) und dem Reinraum (22) zu ermöglichen;
wobei der Wagen (20) Folgendes umfasst:
- eine Unterteilung (24),
- mindestens eine geschützte Tür (28), die ausgelegt ist, um alternativ den Zugang zu der Unterteilung (24) zu ermöglichen und die Unterteilung (24) hermetisch verschließen,
- ein Belüftungssystem (30), und
- erste Schnittstellenmittel (60.1), die die geschützte Tür (28) umgeben;
wobei das Fenster (62) Folgendes umfasst:
- eine Platte (64), die alternativ offen oder dicht verschlossen sein kann, und
- zweite Schnittstellenmittel (60.2), die sich entlang des Umfangs der Platte (64) erstrecken;
**dadurch gekennzeichnet, dass** die ersten Schnittstellenmittel (60.1) und die zweiten Schnittstellenmittel (60.2) zueinander komplementär sind und ermöglichen, dass der Wagen (20) auf reversible Weise fest mit dem Fenster (62) gekoppelt ist, um einen Spalt (68) dazwischen zu definieren, der dicht verschlossen ist.

2. Baugruppe (66) nach Anspruch 1, wobei das Belüftungssystem (30) des Wagens (20) ausgelegt ist, um einen laminaren Luftstrom innerhalb der Unterteilung (24) zu erzeugen.

3. Baugruppe (66) nach Anspruch 1 oder 2, wobei der Wagen (20) ferner ein Biodekontaminationssystem umfasst.

4. Baugruppe (66) nach Anspruch 3, wobei das Biodekontaminationssystem eine Lampe umfasst, die ausgelegt ist, um eine keimtötende Ultraviolettbestrahlung (UVGI) zu emittieren.

5. Baugruppe (66) nach Anspruch 3, wobei das Biodekontaminationssystem eine Hochdruckvorrichtung (32) und eine erste Leitung (34) umfasst, die ausgelegt ist, um einen Strom von Wasserstoffperoxid-basiertem Hochdruckgemisch in die Unterteilung (24) einzuführen.

6. Baugruppe (66) nach Anspruch 5, wobei der Wagen (20) ferner einen oder mehrere Hauptkatalysatoren (58) umfasst, die entlang des Belüftungssystems (30) stromabwärts der Unterteilung (24) angeordnet und dazu ausgelegt sind, die Aufspaltung der Wasserstoffperoxid (H₂O₂) -Moleküle in Wasser (H₂O) und Sauerstoff (O₂) sicherzustellen.

7. Baugruppe (66) nach einem der vorhergehenden Ansprüche, wobei der Wagen (20) eine zweite Leitung (74) umfasst, die das Innere der Unterteilung (24) mit einer Mündung (76) außerhalb der Unterteilung (24) zwischen der geschützten Tür (28) und den ersten Schnittstellenmitteln (60.1) verbindet.

8. Baugruppe (66) nach Anspruch 5 oder 6, wobei der Wagen (20) ferner Folgendes umfasst:
- eine dritte Leitung (84), die den Auslass der Hochdruckvorrichtung (32) mit einem Punkt innerhalb des Belüftungssystems (30) stromabwärts der Hauptkatalysatoren (58) verbindet; und
- ein abnehmbares Zubehörteil (86), das einen Luftverteiler und einen oder mehrere Zusatzkatalysatoren umfasst.

9. Baugruppe (66) nach einem der vorhergehenden Ansprüche, wobei das Fenster (62) ferner eine Mündung (80) zwischen der Platte (64) und den zweiten Schnittstellenmitteln (60.2) umfasst, die mit einem festen Hochdrucksystem (82) verbunden sind.

10. Baugruppe (66) nach Anspruch 9, wobei das Fenster (62) ferner ein zweites Belüftungssystem (78) umfasst, das einen Abgasfilter und feststehende Katalysatoren umfasst.

11. Verfahren zum Übertragen von sauberem Material von einem ersten Reinraum (22) zu einem zweiten Reinraum (22), indem durch einen verschmutzten Bereich (56) hindurchgegangen wird, umfassend die folgenden Schritte:
- Bereitstellen eines Wagens (20) einer Baugruppe (66) nach einem oder mehreren der Ansprüche 1 bis 10 in dem verschmutzten Bereich (56);
- Bereitstellen des ersten Reinraums (22) mit einem ersten Fenster (62) einer Baugruppe (66) nach einem oder mehreren der Ansprüche 1 bis 10;
- Bereitstellen des zweiten Reinraums (22) mit einem zweiten Fenster (62) einer Baugruppe (66) nach einem oder mehreren der Ansprüche 1 bis 10;
- Koppeln des Wagens (20) an das erste Fenster (62) durch die Schnittstellenmittel (60), um einen ersten Spalt (68) zu bilden;
- abdichtendes Schließen des ersten Spalts (68);
- Biodekontaminieren des ersten Spalts (68);
- Eliminieren der Wasserstoffperoxidrückstände aus dem ersten Spalt (68);
- Öffnen der Platte (64) des ersten Fensters (62);
- Öffnen der geschützten Tür (28) des Wagens (20);
- Laden des sauberen Materials in die Unterteilung (24);
- abdichtendes Schließen der geschützten Tür (28);
- abdichtendes Schließen der Platte (64) des ersten Fensters (62);
- Entkoppeln des Wagens (20) von dem ersten Fenster (62) des ersten Reinraums (22);
- Transportieren des Wagens (20) in die Nähe des zweiten Reinraums (22);
- Koppeln des Wagens (20) an das zweite Fenster (62) durch die Schnittstellenmittel (60), um einen zweiten Spalt (68) zu bilden;
- abdichtendes Schließen des zweiten Spalts (68);
- Biodekontaminieren des zweiten Spalts (68);
- Eliminieren der Wasserstoffperoxidrückstände aus dem zweiten Spalt (68);
- Öffnen der Platte (64) des zweiten Fensters (62);
- Öffnen der geschützten Tür (28) des Wagens (20);
- Entladen des sauberen Materials aus der Unterteilung (24);
- abdichtendes Schließen der geschützten Tür (28);
- abdichtendes Schließen der Platte (64) des zweiten Fensters (62); und
- Entkoppeln des Wagens (20) von dem zweiten Fenster (62) des zweiten Reinraums (22).

12. Verfahren zum Übertragen von verschmutztem Material von einem verschmutzten Bereich (56) zu einem Reinraum (22), umfassend die folgenden Schritte:
- Bereitstellen eines Wagens (20) einer Baugruppe (66) nach einem oder mehreren der Ansprüche 1 bis 10 in dem verschmutzten Bereich (56);
- Bereitstellen des Reinraums (22) mit einem Fenster (62) einer Baugruppe (66) nach einem oder mehreren der Ansprüche 1 bis 10;
- Öffnen einer Tür (26, 28) des Wagens (20);
- Laden des verschmutzten Materials in die Unterteilung (24);
- abdichtendes Schließen der Tür (26, 28);
- Koppeln des Wagens (20) an das Fenster (62) des Reinraums (22) durch die Schnittstellenmittel (60), um einen Spalt (68) zu bilden;
- abdichtendes Schließen des Spalts (68);
- Biodekontaminieren des Spalts (68), des Inneren der Unterteilung (24) und des Materials;
- Eliminieren der Wasserstoffperoxidrückstände aus dem Spalt (68) und der Unterteilung (24);
- Öffnen der Platte (64) des Fensters (62);
- Öffnen der geschützten Tür (28) des Wagens (20);
- Entladen des sauberen Materials aus der Unterteilung (24);
- abdichtendes Schließen der geschützten Tür (28);
- abdichtendes Schließen der Platte (64) des Fensters (62); und
- Entkoppeln des Wagens (20) von dem Fenster (62) des Reinraums (22).

13. Verfahren zum Übertragen von verschmutztem Material von einem verschmutzten Bereich (56) zu einem Reinraum (22), umfassend die folgenden Schritte:
- Bereitstellen eines Wagens (20) einer Baugruppe (66) nach einem oder mehreren der Ansprüche 1 bis 10 in dem Reinraum (22);
- Bereitstellen des Reinraums (22) mit einem Fenster (62) einer Baugruppe (66) nach einem oder mehreren der Ansprüche 1 bis 10;
- Koppeln des Wagens (20) an das Fenster (62) des Reinraums (22) durch die Schnittstellenmittel (60), um einen Spalt (68) zu bilden;
- abdichtendes Schließen des Spalts (68);
- Öffnen der Platte (64) des Fensters (62);
- Öffnen der geschützten Tür (28) des Wagens (20);
- Laden des verschmutzten Materials in die Unterteilung (24);
- Schließen der geschützten Tür (28) des Wagens (20);
- abdichtendes Schließen der Platte (64) des Fensters (62);
- Biodekontaminieren des Inneren der Unterteilung (24), des Materials und des Spalts (68);
- Eliminieren der Wasserstoffperoxidrückstände aus der Unterteilung (24) und dem Spalt (68);
- Entkoppeln des Wagens (20) vom Fenster (62);
- Bewegen des Wagens (20) innerhalb des Reinraums (22);
- Öffnen einer Tür (26, 28) des Wagens (20); und
- Entladen des sauberen Materials aus der Unterteilung (24) .

## Revendications

1. Ensemble chariot-fenêtre (66), comprenant un chariot (20) pour déplacer du matériel vers/depuis une salle blanche (22) et au moins une fenêtre (62) adaptée pour permettre la communication entre un espace sale (56) et ladite salle blanche (22) ;
dans lequel ledit chariot (20) comprend :
- un compartiment (24),
- au moins une porte protégée (28) adaptée pour permettre alternativement l'accès au compartiment (24) et la fermeture hermétique du compartiment (24),
- un système de ventilation (30), et
- des premiers moyens d'interface (60.1) entourant la porte protégée (28) ;
dans lequel ladite fenêtre (62) comprend :
- un panneau (64) qui peut alternativement être ouvert ou fermé hermétiquement et
- des deuxièmes moyens d'interface (60.2) s'étendant le long de la périphérie du panneau (64) ;
**caractérisé en ce que** les premiers moyens d'interface (60.1) et les deuxièmes moyens d'interface (60.2) sont mutuellement complémentaires et permettent au chariot (20) d'être fermement accouplé à la fenêtre (62) d'une manière réversible, de sorte à définir un espacement (68) entre eux qui est fermé hermétiquement.

2. Ensemble (66) selon la revendication 1, dans lequel le système de ventilation (30) du chariot (20) est adapté pour créer un flux d'air laminaire à l'intérieur du compartiment (24).

3. Ensemble (66) selon la revendication 1 ou 2, dans lequel le chariot (20) comprend de plus un système de décontamination biologique.

4. Ensemble (66) selon la revendication 3, dans lequel le système de décontamination biologique comprend une lampe adaptée pour émettre une irradiation germicide ultraviolette (UVGI).

5. Ensemble (66) selon la revendication 3, dans lequel le système de décontamination biologique comprend un appareil HP (32) et un premier conduit (34) adapté pour introduire un flux de mélange HP à base de peroxyde d'hydrogène dans le compartiment (24).

6. Ensemble (66) selon la revendication 5, dans lequel le chariot (20) comprend de plus un ou plusieurs catalyseurs principaux (58) placés le long du système de ventilation (30), en aval du compartiment (24) et adaptés pour assurer la scission des molécules de peroxyde d'hydrogène (H₂O₂) en eau (H₂O) et en oxygène (O₂).

7. Ensemble (66) selon l'une quelconque des revendications précédentes, dans lequel le chariot (20) comprend un deuxième conduit (74) reliant l'intérieur du compartiment (24) à une embouchure (76) à l'extérieur du compartiment (24), entre la porte protégée (28) et les premiers moyens d'interface (60.1).

8. Ensemble (66) selon la revendication 5 ou 6, dans lequel le chariot (20) comprend de plus :
- un troisième conduit (84) reliant la sortie de l'appareil HP (32) à un point situé à l'intérieur du système de ventilation (30), en aval des catalyseurs principaux (58) ; et
- un accessoire amovible (86), comprenant un collecteur d'air et un ou plusieurs catalyseurs auxiliaires.

9. Ensemble (66) selon l'une quelconque des revendications précédentes, dans lequel la fenêtre (62) comprend de plus une embouchure (80), entre le panneau (64) et les deuxièmes moyens d'interface (60.2), reliée à un système HP (82) fixe.

10. Ensemble (66) selon la revendication 9, dans lequel la fenêtre (62) comprend de plus un deuxième système de ventilation (78), comprenant un filtre d'échappement et des catalyseurs fixes.

11. Procédé de transfert de matériel propre d'une première salle blanche (22) à une seconde salle blanche (22) en passant par un espace sale (56), comprenant les étapes de :
- prévoir un chariot (20) d'un ensemble (66) selon l'une ou plusieurs des revendications 1 à 10 dans l'espace sale (56) ;
- équiper la première salle blanche (22) d'une première fenêtre (62) d'un ensemble (66) selon l'une ou plusieurs des revendications 1 à 10 ;
- équiper la deuxième salle blanche (22) d'une deuxième fenêtre (62) d'un ensemble (66) selon l'une ou plusieurs des revendications 1 à 10 ;
- accoupler le chariot (20) à la première fenêtre (62) par les moyens d'interface (60) de manière à former un premier espacement (68) ;
- fermer hermétiquement le premier espacement (68) ;
- décontaminer biologiquement le premier espacement (68) ;
- éliminer les résidus de peroxyde d'hydrogène du premier espacement (68) ;
- ouvrir le panneau (64) de la première fenêtre (62) ;
- ouvrir la porte protégée (28) du chariot (20) ;
- charger le matériel propre dans le compartiment (24) ;
- fermer hermétiquement la porte protégée (28) ;
- fermer hermétiquement le panneau (64) de la première fenêtre (62) ;
- désaccoupler le chariot (20) de la première fenêtre (62) de la première salle blanche (22) ;
- transporter le chariot (20) à proximité de la deuxième salle blanche (22) ;
- accoupler le chariot (20) à la deuxième fenêtre (62) par les moyens d'interface (60) de manière à former un deuxième espacement (68) ;
- fermer hermétiquement le deuxième espacement (68) ;
- décontaminer biologiquement le deuxième espacement (68) ;
- éliminer les résidus de peroxyde d'hydrogène du deuxième espacement (68) ;
- ouvrir le panneau (64) de la deuxième fenêtre (62) ;
- ouvrir la porte protégée (28) du chariot (20) ;
- décharger le matériel propre du compartiment (24) ;
- fermer hermétiquement la porte protégée (28) ;
- fermer hermétiquement le panneau (64) de la deuxième fenêtre (62) ; et
- désaccoupler le chariot (20) de la deuxième fenêtre (62) de la deuxième salle blanche (22).

12. Procédé de transfert de matériel sale d'un espace sale (56) vers une salle blanche (22), comprenant les étapes de :
- prévoir un chariot (20) d'un ensemble (66) selon l'une ou plusieurs des revendications 1 à 10 dans l'espace sale (56) ;
- équiper la salle blanche (22) d'une fenêtre (62) d'un ensemble (66) selon l'une ou plusieurs des revendications 1 à 10 ;
- ouvrir une porte (26, 28) du chariot (20) ;
- charger le matériel sale dans le compartiment (24) ;
- fermer hermétiquement la porte (26, 28) ;
- accoupler le chariot (20) à la fenêtre (62) de la salle blanche (22) par les moyens d'interface (60) de manière à former un espacement (68) ;
- fermer hermétiquement l'espacement (68) ;
- décontaminer biologiquement l'espacement (68), l'intérieur du compartiment (24) et le matériel ;
- éliminer les résidus de peroxyde d'hydrogène de l'espacement (68) et du compartiment (24) ;
- ouvrir le panneau (64) de la fenêtre (62) ;
- ouvrir la porte protégée (28) du chariot (20) ;
- décharger le matériel propre du compartiment (24) ;
- fermer hermétiquement la porte protégée (28) ;
- fermer hermétiquement le panneau (64) de la fenêtre (62) ; et
- désaccoupler le chariot (20) de la fenêtre (62) de la salle blanche (22).

13. Procédé de transfert de matériel sale d'un espace sale (56) vers une salle blanche (22), comprenant les étapes de :
- prévoir un chariot (20) d'un ensemble (66) selon l'une ou plusieurs des revendications 1 à 10 dans la salle blanche (22) ;
- équiper la salle blanche (22) d'une fenêtre (62) d'un ensemble (66) selon l'une ou plusieurs des revendications 1 à 10 ;
- accoupler le chariot (20) à la fenêtre (62) de la salle blanche (22) par les moyens d'interface (60) de manière à former un espacement (68) ;
- fermer hermétiquement l'espacement (68) ;
- ouvrir le panneau (64) de la fenêtre (62) ;
- ouvrir la porte protégée (28) du chariot (20) ;
- charger le matériel sale dans le compartiment (24) ;
- fermer la porte protégée (28) du chariot (20) ;
- fermer hermétiquement le panneau (64) de la fenêtre (62) ;
- décontaminer biologiquement l'intérieur du compartiment (24), le matériel et l'espacement (68) ;
- éliminer les résidus de peroxyde d'hydrogène du compartiment (24) et de l'espacement (68) ;
- désaccoupler le chariot (20) de la fenêtre (62) ;
- déplacer le chariot (20) à l'intérieur de la salle blanche (22) ;
- ouvrir une porte (26, 28) du chariot (20) ; et
- décharger le matériel propre du compartiment (24).
